# EUROPÄISCHE PATENTSCHRIFT

(11) **EP 3 595 577 B1**
(45) Veröffentlichungstag und Bekanntmachung des Hinweises auf die Patenterteilung: **07.04.2021**
(21) Anmeldenummer: 18709579.9
(22) Anmeldetag: 08.03.2018
(51) Int. Cl.: A61C 13/30

(54) **ENDODONTIESTIFT**
ENDODONTIC PIN
POINTE ENDODONTIQUE

(30) Priorität: 14.03.2017 AT 982017
(43) Veröffentlichungstag der Anmeldung: 22.01.2020
(73) Patentinhaber: edelweiss dentistry products gmbh, 6922 Wolfurt (AT)
(72) Erfinder: LAMPL Stephan, 9451 Kriessern (CH)
(74) Vertreter: Fechner, Thomas
(86) Internationale Anmeldenummer: PCT/EP2018/055724
(87) Internationale Veröffentlichungsnummer: WO 2018/166879

(56) Entgegenhaltungen:
- EP-A1- 1 925 269
- EP-A1- 3 090 701
- DE-U1-202010 000 297
- JP-A- 2009 118 914
- US-A1- 2003 113 691
- US-A1- 2005 003 328
- US-B1- 6 183 253

## Beschreibung

Die vorliegende Erfindung betrifft einen Endodontiestift gemäß des Oberbegriffs des Patentanspruchs 1.

In der Endodontie, einem Teilbereich der Zahnheilkunde, wird ein verbleibender Rest eines natürlichen Zahnes bzw. zumindest einer natürlichen Zahnwurzel dazu genutzt, um einen Wurzelstift bzw. Endodontiestift mit seinem Wurzelbereich in der Zahnwurzel zu befestigen. Der Kronenbereich des Endodontiestiftes wird dazu benutzt, einen verbliebenen Rest einer natürlichen Zahnkrone zu stabilisieren und/oder ansonsten zu ergänzen und/oder um eine Zahnkronenverblendung am Kronenbereich des Endodontiestiftes zu befestigen.

Beim Stand der Technik sind Endodontiestifte aus verschiedenen Materialien bekannt. So gibt es verschiedenste Endodontiestifte aus Metall, wie z.B. Titan und dergleichen. Problematisch sind hier zum einen die doch deutlich von der natürlichen Zahnsubstanz abweichenden mechanischen Eigenschaften dieser Metalle. Zum anderen gibt es auch Probleme bei der Biokompatibilität von Metallen, da Einlagerungen von Metallionen im Zahn und/oder im umliegenden Weichgewebe mittlerweile kritisch in Frage gestellt werden.

Beim Stand der Technik sind auch Endodontiestifte bekannt, bei denen in das organische Bindemittel Glas- oder Karbonfasern eingebettet sind. Die Erfahrung zeigt aber, dass bei diesen faserverstärkten Kunststoffen Ermüdungserscheinungen und vor allem ein Separieren von Fasern und Bindemittel auftreten können.

Ein gattungsgemäßer Endodontiestift ist aus der US 2003/0113691 A1 bekannt.

Aufgabe der Erfindung ist es, einen Endodontiestift der oben genannten Art dahingehend zu verbessern, dass er in seinen mechanischen Eigenschaften der natürlichen Zahnsubstanz möglichst nahe kommt und dabei möglichst langlebig ist.

Hierfür sieht die Erfindung einen Endodontiestift gemäß des Patentanspruchs 1 vor.

Bei der Erfindung ist somit vorgesehen, zur Herstellung des Endodontiestiftes ein zusammengesetztes Material bzw. in anderen Worten ein Composite zu verwenden, welches einerseits ein organisches Bindemittel aufweist und andererseits mit Glaskörnern als Füllstoff gefüllt ist. Es hat sich gezeigt, dass hierdurch Endodontiestifte geschaffen werden können, welche in ihren mechanischen Eigenschaften der natürlichen Zahnsubstanz sehr nahe kommen und auch eine ausreichende Langlebigkeit aufweisen. Bevorzugte Varianten von erfindungsgemäßen Endodontiestiften weisen als fertiges Endprodukt eine Biegefestigkeit im Bereich von 150 MPa (MegaPascal) bis 250 MPa, vorzugsweise von 180 MPa bis 220 MPa, besonders bevorzugt von 200 MPa auf. Die Druckfestigkeit solcher Endodontiestifte liegt günstigerweise im Bereich von 450 MPa bis 550 MPa, bevorzugt im Bereich von 500 bis 550 MPa, besonders bevorzugt bei 550 MPa. Das Biegemodul solcher Endodontiestifte liegt günstigerweise im Bereich von 12 GPa (GigaPascal) bis 25 GPa, vorzugsweise von 17 GPa bis 25 GPa, besonders bevorzugt bei 20 GPa. Die Oberflächenhärte bevorzugter Endodontiestifte liegt günstigerweise im Bereich von 85 HV (Vickershärte) bis 120 HV, vorzugsweise von 90 HV bis 100 HV, besonders bevorzugt bei 95 HV. All diese Parameter können gemäß EN ISO 4049; Ausgabe: 2010-03-1 bestimmt werden.

Die Korngrößen der Glaskörner des zusammengesetzten Materials liegen bevorzugt im Bereich von 0,01 µm (Micrometer) bis 50µm, besonders bevorzugt im Bereich von 0,01µm bis 3µm. Die Glaskörner können gerundet sein, aber auch eine davon abweichende Kornform haben. Im Sinne einer möglichsten dichten Packung weist das zusammengesetzte Material bevorzugt Glaskörner mit unterschiedlichen Korngrößen auf. Das zusammengesetzte Material kann Glaskörner einer einzigen Glasart aber auch von verschiedenen Glasarten aufweisen. Es handelt sich günstigerweise um sogenannte hochgefüllte zusammengesetzte Materialien bzw. Composites. Der Volumenanteil der Glaskörner im fertigen Endprodukt des Endodontiestifts bzw. Grundstifts beträgt günstigerweise zumindest 78 Vol.-%, bevorzugt zumindest 83 Vol.-%, besonders bevorzugt zumindest 90 Vol.-%. Bei den Glaskörnern handelt es sich bevorzugt um solche, welche Bariumglas und/oder Strontiumglas aufweisen oder daraus bestehen. Günstigerweise haben die Glaskörner eine silanisierte Oberfläche. Insbesondere bei Bariumglas und Strontiumglas handelt es sich um Werkstoffe, welche von sich röntgenopak sind, sodass der Endodontiestift auch nach Applikation im Zahn des Patienten mittels eines Röntgenbildes abbildbar ist. Sind die Glaskörner selbst nicht röntgenopak, so kann dem zusammengesetzten Material als weiterer Füllstoff auch Aluminiumoxid oder dergleichen zugesetzt werden, um eine entsprechende Röntgenopazität und damit Sichtbarkeit in einem Röntgenbild zu verschaffen. Als weiterer Füllstoff kann dem zusammengesetzten Material des Endodontiestiftes Zinkoxid (ZnO) zugesetzt sein, um eine antibakterielle Wirkung zu erzielen. Günstigerweise liegt der Volumenanteil des Zinkoxids im Endodontiestift, also im Endprodukt, bei ca. 2-5 %. Weitere Füllstoffe des zusammengesetzten Materials können Pigmente also Farbstoffe und/oder Katalysatoren zur Unterstützung der Polymerisierung des organischen Bindemittels und/oder sonstige Additive sein.

Das organische Bindemittel weist in bevorzugten Ausführungsvarianten der Erfindung zumindest ein Methacrylat auf oder besteht daraus. Bevorzugt weist das organische Bindemittel Methacrylat zumindest als Hauptbestandteil auf.

Als zusammengesetztes Material für die Herstellung des Grundstiftes bzw. des Endodontiestiftes kann z.B. das unter der Bezeichnung REF 2061 erhältliche Produkt der Firma Indigodental GmbH & Co. KG, in Pinneberg, Deutschland verwendet werden. Dieses Produkt enthält Triethylenglycoldimethacrylat, Urethandimethacrylat, Bis -GMA und ethoxyliertes Bisphenol A Dimethacrylat. Als organisches Bindemittel kann z.B. das Produkt REF 2050 dieser Firma verwendet werden.

Bei einem Endodontiestift handelt es sich um einen Stift, welcher in der Endodontie eingesetzt werden kann. Ein erfindungsgemäßer Endodontiestift kann auch als Wurzelstift bezeichnet werden. Der erfindungsgemäße Endodontiestift kann ausschließlich aus dem Grundstift bestehen. Erfindungsgemäße Endodontiestifte können aber auch weitere Bestandteile wie z.B. den weiter unten noch genannten Mantelaufbaukörper aufweisen. Der Grundstift hat jedenfalls einen Wurzelbereich. Dies ist der Teil des Grundstiftes, welcher zur Befestigung des Grundstiftes in einer natürlichen Zahnwurzel verankert wird. Hierzu wird in der Regel in der natürlichen Zahnwurzel ein entsprechender Kanal z.B. durch Bohren geschaffen, in den der Wurzelbereich des Grundstiftes eingesetzt wird. Der Grundstift weist zusätzlich auch einen Kronenbereich auf. Dies ist der Teil, der dazu dient, im Bereich einer natürlichen Zahnkrone eines natürlichen Zahnes bzw. meist eines verbliebenen Restes davon, angeordnet zu werden. Der Kronenbereich kann aber auch zur Befestigung einer Zahnkronenverblendung dienen, insbesondere dann, wenn von der natürlichen Zahnkrone gar nichts mehr oder nur noch Reste vorhanden sind. Erfindungsgemäße Endodontiestifte können also einerseits dazu verwendet werden, um eine Zahnkronenverblendung, also eine künstlich geschaffene Zahnkrone oder einen künstlich geschaffenen Teil einer Zahnkrone an einer natürlichen Zahnwurzel zu befestigen. Erfindungsgemäße Endodontiestifte können aber auch dazu verwendet werden, einen verbliebenen Teil der natürlichen Zahnkrone zu stabilisieren und als Verankerung für gegebenenfalls zu ergänzende künstliche Teile der Zahnkrone dienen. In diesem Sinne kann somit die Zahnkronenverblendung nur Teile einer natürlichen Zahnkrone oder aber auch die gesamte natürliche Zahnkrone ersetzen. Um den Wurzelbereich des Grundstiftes in der Zahnwurzel zu befestigen, wird häufig der in der natürlichen Zahnwurzel ursprünglich vorhandene Nervenkanal, meist ein Stück weit aufgebohrt, als Kanal genutzt, um den Wurzelbereich in diesem Kanal zu befestigen.

Bei erfindungsgemäßen Endodontiestiften bzw. Wurzelstiften handelt es sich bevorzugt um ein vorkonfektioniertes Massenprodukt, welches kostengünstig hergestellt und in einer einzigen Sitzung vom Zahnarzt in der verbliebenen Zahnwurzel bzw. im verbliebenen natürlichen Restzahn appliziert werden kann. Da das zusammengesetzte Material des erfindungsgemäßen Endodontiestiftes in seinen mechanischen Eigenschaften der natürlichen Zahnsubstanz sehr nahe kommt, können für ein vor der Applikation gegebenenfalls notwendiges Einkürzen oder einer entsprechenden Formanpassung die beim Zahnarzt üblichen Bohrer, Schleifer und dergleichen verwendet werden.

Der Wurzelbereich des Grundstiftes wird bevorzugt mit einem Befestigungsmaterial oder in anderen Worten einem Zement in den vorab fertig präparierten und gereinigten Kanal der natürlichen Zahnwurzel eingeklebt bzw. einzementiert. Das hierzu verwendete Befestigungsmaterial weist bevorzugt eine ähnliche Zusammensetzung wie das zusammengesetzte Material des Grundstiftes auf. Das Befestigungsmaterial ist günstigerweise somit auch ein zusammengesetztes Material mit einem organischen Bindemittel und Glaskörnern als Füllstoff. Das organische Bindemittel des Befestigungsmaterials kann also bevorzugt wiederum zumindest ein Methacrylat aufweisen oder daraus bestehen. Da das Befestigungsmaterial im nicht ausgehärteten Zustand aber flüssig bzw. zumindest pastös sein sollte, ist günstigerweise vorgesehen, dass der Füllgrad, also der Volumenanteil der Füllstoffe, insbesondere der Glaskörner, geringer ist als beim zusammengesetzten Material des Endodontiestiftes. Der Füllstoffanteil bzw. insbesondere der Anteil an Glaskörnern beträgt beim Befestigungsmaterial günstigerweise zwischen 45 Vol.-% und 65 Vol.-%.

Aufgrund des organischen Bindemittelanteils des Befestigungsmaterials kann dieses durch Lichteinwirkung polymerisiert und damit ausgehärtet werden. Um eine möglichst gute und dauerhafte Verbindung und ein schnelles Aushärten des Befestigungsmaterials zu unterstützen und zu forcieren, sehen besonders bevorzugte Varianten erfindungsgemäßer Endodontiestifte vor, dass der Grundstift im Kronenbereich eine Lichteinspeisefläche aufweist und zur Weiterleitung von über die Lichteinspeisefläche eingespeistem Licht in den Wurzelbereich zumindest bereichsweise, vorzugsweise vollständig, lichtleitend ausgebildet ist. In diesen Ausgestaltungsformen wird der Grundstift somit selbst als eine Art Lichtleiter verwendet, sodass über die Lichteinspeisefläche in den Grundstift eingespeistes Licht über den Grundstift selbst zum Befestigungsmaterial geleitet wird, um das Aushärten des Befestigungsmaterials zu forcieren.

Um entsprechend gut lichtleitend ausgebildet zu sein, weist der Grundstift in bevorzugten Ausgestaltungsformen zumindest bereichsweise, vorzugsweise vollständig, eine relativ geringe Opazität also anders herum ausgedrückt eine relativ hohe Transluzenz auf. Bevorzugt liegt die Opazität des Grundstiftes zumindest bereichsweise, vorzugsweise vollständig, in einem Bereich zwischen 15% und 45%, bevorzugt in einem Bereich von 25% bis 30%, besonders bevorzugt bei ca. 27%. Diese Opazität, wie auch alle nachfolgend genannten Opazitäten können nach DIN 6174 bestimmt werden. Die Lichteinspeisefläche befindet sich günstigerweise am, dem Wurzelbereich gegenüberliegenden Ende des Kronenbereiches des Grundstiftes.

Um das über die Lichteinspeisefläche eingespeiste Licht besonders gut dorthin zu lenken, wo es zum Aushärten des Befestigungs- bzw. Zementmaterials benötigt wird, sehen bevorzugte Varianten vor, dass der Grundstift einen Linsenkörper aufweist. Dieser Linsenkörper befindet sich günstigerweise im Kronenbereich des Grundstiftes. Vorzugsweise ist der Linsenkörper als eine Verdickung des Grundstiftes ausgebildet. Dies bedeutet, der Linsenkörper weist also bevorzugt einen größeren Durchmesser sowohl als der Wurzelbereich als auch als der restliche Kronenbereich des Grundstiftes auf. Der Linsenkörper ist an seiner lichtbrechenden Wirkung zu erkennen. Er lenkt das über die Lichteinspeisefläche eingespeiste Licht so ab, dass es dort hin geleitet wird, wo es zum Aushärten des Befestigungsmaterials benötigt wird. Dies sind insbesondere die Außenflächen des Wurzelbereichs des Grundstiftes wie auch gegebenenfalls vorhandene weiter unten noch im Detail erläuterte Schulterbereiche des Endodontiestiftes, welche ebenfalls an der verbliebenen natürlichen Zahnwurzel anliegen können.

In bevorzugten Ausgestaltungsformen ist der Wurzelbereich des Grundstiftes als ein sich in Richtung weg vom Kronenbereich verjüngender Konus ausgebildet. Bevorzugt handelt es sich um einen bezüglich einer Längsachse des Grundstiftes rotationssymmetrischen Wurzelbereich. Der Konus ist günstigerweise somit kegelstumpf bzw. kegelförmig. Die Außenfläche des Wurzelbereichs des Grundstiftes kann eine gewisse Rauigkeit aufweisen. Bevorzugt sind aber keine von außen sichtbaren Strukturen wie Gewinde oder dergleichen in der Außenfläche des Wurzelbereichs des Grundstiftes vorhanden. Bei bevorzugten Ausgestaltungsformen könnte man also davon sprechen, dass die äußere Oberfläche des Wurzelbereichs glatt ausgebildet ist.

Erfindungsgemäße Endodontiestifte können, wie gesagt, ausschließlich aus dem Grundstift bestehen. Andere Ausgestaltungsformen der Erfindung können aber auch vorsehen, dass der Endodontiestift zusätzlich zum Grundstift einen Mantelaufbaukörper aufweist, welcher, vorzugsweise ausschließlich, den Kronenbereich des Grundstiftes, vorzugsweise in sich umfangsgeschlossen, ummantelt. Vorzugsweise ist dabei vorgesehen, dass der Mantelaufbaukörper eine höhere Opazität als der Grundstift aufweist. Die höhere Opazität des Mantelaufbaukörpers gegenüber dem Grundstift dient dazu, dass die letztendlich vollständig oder teilweise geschaffene Krone dem natürlichen Erscheinungsbild einer Zahnkrone auch farblich möglichst nahe kommt. Die Opazität des Mantelaufbaukörpers liegt günstigerweise im Bereich von 50% bis 80%, vorzugsweise von 65% bis 75%, besonders bevorzugt bei ca. 70%.

Der Mantelaufbaukörper kann, abgesehen von zur Einfärbung benötigten Pigmenten also Farbstoffen, aus demselben zusammengesetzten Material wie der Grundstift bestehen oder ein solches zumindest aufweisen. Zum Einfärben des zur Herstellung des Mantelaufbaukörpers verwendeten zusammengesetzten Materials können an sich bekannte organische und/oder auch anorganische Pigmente verwendet werden, z.B. auch Weißmacher wie TIO2. Die Pigmente können so abgestimmt werden, dass das Gesamterscheinungsbild des Mantelaufbaukörpers dem des natürlichen Dentins im Mund des Patienten möglichst nahe kommt. Im Lab-Farbraum ausgedrückt, hat der Mantelaufbaukörper bevorzugt einen L-Wert von 59 bis 69 und/oder einen a-Wert von -0,35 bis -3,2 und/oder einen b-Wert von -0,4 bis - 6,95. Die Bestimmung des L-Wertes, des a-Wertes und des b-Wertes kann, wie die Bestimmung der Opazität, gemäß den Vorgaben von DIN 6174 erfolgen. Bevorzugte Ausgestaltungsformen solcher Endodontiestifte sehen vor, dass der Mantelaufbaukörper, welcher auch als restaurativer Mantelaufbau bezeichnet werden könnte, einen gegenüber dem Wurzelbereich des Grundstiftes hervorspringenden Schulterbereich des Endodontiestiftes ausbildet. Dieser Schulterbereich ist günstigerweise umfangsgeschlossen um den gesamten Endodontiestift ausgebildet. Mit diesem Schulterbereich kann sich der Endodontiestift auf der verbliebenen natürlichen Zahnwurzel abstützen.

Bevorzugte Ausgestaltungsformen der Erfindung sehen vor, dass der Grundstift in sich monolithisch ausgebildet ist. Bevorzugt gilt dies auch für den gegebenenfalls vorhandenen Mantelaufbaukörper. Monolithisch bedeutet dabei aus einem Guss bzw. einstückig hergestellt. Im Grundstift und gegebenenfalls auch im Mantelaufbaukörper befinden sich also günstigerweise keine Grenzflächen. Der Grundstift besteht also bevorzugt aus einem Stück und das zusammengesetzte Material ist innerhalb des Grundstiftes bevorzugt überall gleich ausgebildet. Das Gleiche gilt bevorzugt auch für den Mantelaufbaukörper.

Erfindungsgemäße Endodontiestifte sehen vor, dass die Glaskörner miteinander verschmolzen sind. Die Glaskörner des zusammengesetzten Materials des Endodontiestiftes sind somit in entsprechenden mikroskopischen Aufnahmen noch als solche zu erkennen aber durch Aufschmelzen und miteinander Verschmelzen ihrer Oberflächen miteinander verbunden. Dieses miteinander Verschmelzen der Glaskörner kann durch eine Laserbehandlung des Grundstiftes und gegebenenfalls auch des Mantelaufbaukörpers erreicht werden, wie dies weiter unten noch im Detail erläutert wird. Der Grundstift und/oder auch der gegebenenfalls vorhandene Mantelaufbaukörper können somit laserbehandelt sein, was an dem Verschmelzen der Glaskörner miteinander zu erkennen ist. Durch das miteinander Verschmelzen wird noch ein stärkerer Verbund erzielt, als der der durch die Verbindung der Glaskörner mittels des ausgehärteten organischen Bindemittels vorab schon vorhanden ist. In bevorzugten Ausgestaltungsformen sind die Glaskörner im gesamten Grundstift miteinander verschmolzen. Soweit ein Mantelaufbaukörper vorhanden ist, gilt dies in bevorzugten Ausgestaltungsformen auch für den Mantelaufbaukörper. Es sind also günstigerweise auch die Glaskörner im gesamten gegebenenfalls vorhandenen Mantelaufbaukörper miteinander verschmolzen.

Um in der verbleibenden natürlichen Zahnwurzel einen für den Wurzelbereich des Grundstiftes passenden Kanal schaffen zu können, werden günstigerweise Bohrer zur Verfügung gestellt, die in ihrer Form an die Form des Wurzelbereichs des Grundstifts angepasst sind. Angepasst bedeutet dabei, dass die Bohrer in die verbleibende natürlich Zahnwurzel ein Loch bzw. einen Kanal bohren, welches bzw. welcher den Raum schafft, der benötigt wird, um darin den Wurzelbereich des Grundstiftes samt benötigtem Befestigungsmaterial zu befestigen. Die Form des Bohrers entspricht also im Wesentlichen der Form des Wurzelbereichs des Grundstifts bzw. kann geringfügig größer sein, um noch Raum für das Befestigungsmaterial zu schaffen. In diesem Zusammenhang betrifft die Erfindung auch ein Set mit zumindest einem, vorzugsweise mehreren, erfindungsgemäßen Endodontiestiften und zumindest einem Bohrer, wobei die Form des Bohrers an die Form des Wurzelbereichs des Grundstifts des Endodontiestifts angepasst ist. Ein solches Set kann natürlich auch mehrere Endodontiestifte und soweit notwendig mehrere dazu passende Bohrer mit umfassen. Zusätzlich kann das Set auch das passende Befestigungsmaterial mit umfassen. Die Bohrer können einen Längsanschlag und/oder eine, vorzugsweise sichtbare, Markierung aufweisen, damit das Bohrloch immer in der optimalen Länge bzw. Tiefe in die Zahnwurzel eingebohrt wird.

Weitere Merkmale und Einzelheiten bevorzugter Ausgestaltungsformen erfindungsgemäßer Endodontiestifte sowie von Verfahren zu deren Herstellung werden nachfolgend in der Figurenbeschreibung beispielhaft erläutert. Es zeigen:
Fig. 1 und 2 zwei Seitenansichten aus zwei um 90° gegeneinander gedrehten Richtungen auf einen erfindungsgemäßen Grundstift;
Fig. 3 bis 7 verschiedene Ausführungsbeispiele von erfindungsgemäßen Endodontiestiften mit einem Grundstift gemäß der Fig. 1 und 2 und verschiedenen Mantelaufbaukörpern;
Fig. 8 bis 13 Beispiele der Applikation der Endodontiestifte aus den Fig. 1 bis 7 in Zähnen;
Fig. 14 eine schematisierte vergrößerte Darstellung zum zusammengesetzten Material des erfindungsgemäßen Endodontiestiftes;
Fig. 15 und 16 Darstellungen zu Herstellverfahren und
Fig. 17 eine schematisierte Darstellung eines Bohrers mit an den Grundstift angepasster Form.

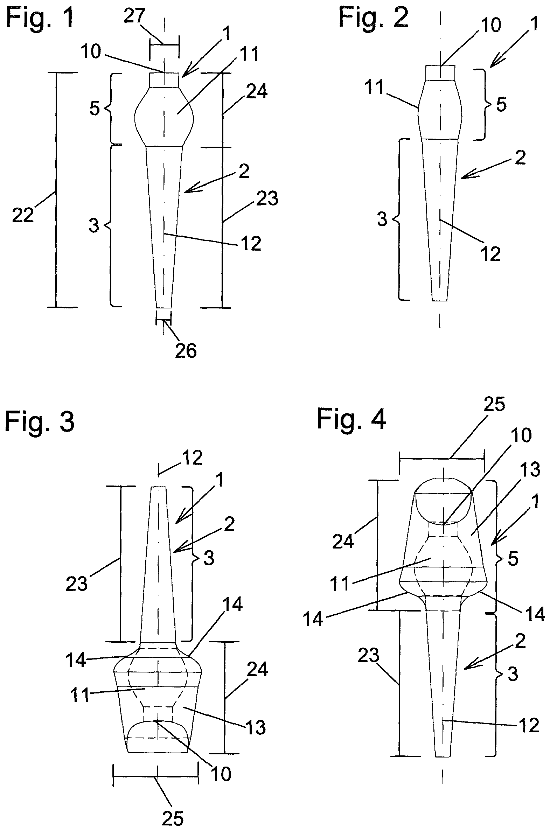

Das in den Fig. 1 und 2 dargestellte Beispiel eines erfindungsgemäßen Endodontiestifts 1 besteht ausschließlich aus dem Grundstift 2. Der Grundstift 2 weist einen Wurzelbereich 3 sowie einen Kronenbereich 5 auf. Der Wurzelbereich 3 ist dazu vorgesehen, in einem entsprechenden Kanal 33 in einer von einem natürlichen Zahn verbliebenen Zahnwurzel 4 befestigt zu werden. Der Kronenbereich 5 des Grundstiftes 2 dient zur Anordnung in einer natürlichen Zahnkrone oder wenn diese nicht mehr oder nur noch zum Teil vorhanden ist, zur Befestigung einer Zahnkronenverblendung 6 am Endodontiestift 1 bzw. am Grundstift 2. Der Wurzelbereich 3 ist als sich in Richtung weg vom Kronenbereich 5 verjüngender Konus ausgebildet und bezüglich der Längsachse 12 des Grundstiftes 2 rotationssymmetrisch ausgebildet. Der Kronenbereich 5 des Grundstiftes 2 kann ebenfalls rotationssymmetrisch bezüglich der Längsachse 12 des Grundstiftes 2 ausgebildet sein. Im gezeigten Ausführungsbeispiel ist er dies nicht, wie der Vergleich der Fig. 1 und 2 zeigt. In Fig. 2 ist die Seitensicht aus einer um 90° gegen Fig. 1 gedrehten Richtung dargestellt. Der Kronenbereich 5 endet in diesen Ausführungsbeispielen auf seiner, dem Wurzelbereich 3 entgegengesetzten Seite in der Lichteinspeisungsfläche 10. Im Kronenbereich 5 befindet sich in diesem Ausführungsbeispiel auch die Verdickung 11, welche einen Linsenkörper ausbildet. In den bevorzugten Ausgestaltungsformen, wie den hier dargestellten, ist der gesamte Grundstift 2 so transparent bzw. transluzent ausgebildet, dass er lichtleitend ist. Hierdurch wird erreicht, dass über die Lichteinspeisefläche 10 eingespeistes Licht innerhalb des Grundstiftes 2 bis in den Wurzelbereich 3 geleitet wird. Die als Linse fungierende Verdickung 11 bricht dabei das Licht in den Wurzelbereich 3 hinein und verstärkt damit die lichtleitende Wirkung. Die durch die Verdickung 11 gebildete Linse kann auch so ausgebildet sein, dass sie das Licht nicht nur in den Wurzelbereich 3 sondern auch in den Schulterbereich 14 der in den Fig. 3 bis 7 gezeigten Ausführungsvarianten von Endodontiestiften 1 leitet. Diese lichtleitende Ausbildung des Grundstiftes 2 ermöglicht ein besonders gutes und schnelles Aushärten von Befestigungsmaterial 28, mit dem der Wurzelbereich 3 des Grundstiftes 2 in der Zahnwurzel 4 befestigt wird. Der gesamte Grundstift 2 ist monolithisch, also aus einem Guss, ausgebildet. Er besteht erfindungsgemäß aus einem zusammengesetzten Material 7, welches zumindest ein organisches Bindemittel 8 und Glaskörner 9 als Füllstoff aufweist. Darüber hinaus kann das zusammengesetzte Material 7 die eingangs bereits genannten sonstigen Füllstoffe zusätzlich mit aufweisen. Die Glaskörner 9 sind miteinander verschmolzen und damit einerseits durch das zwischen ihnen angeordnete organische Bindemittel 8 und andererseits durch das miteinander Verschmelzen fest und dauerhaft miteinander verbunden. Es wird ein Aufbau des Grundstiftes 2 erreicht, der die besonders günstigen, eingangs bereits dargelegten mechanischen Eigenschaften aufweist.

In den Fig. 3 bis 7 sind Ausführungsbeispiele erfindungsgemäßer Endodontiestifte 1 gezeigt, bei denen die Endodontiestifte 1 jeweils einen Grundstift 2 und einen Mantelaufbaukörper 13 aufweisen. In allen diesen Ausführungsbeispielen ummanteln die jeweiligen Mantelaufbaukörper 13 den Kronenbereich 5 des jeweiligen Grundstiftes 2 umfangsgeschlossen. Der Mantelaufbaukörper 13 hat in den bevorzugten Ausgestaltungsformen jeweils eine höhere Opazität als der Grundstift 2. Jeder dieser Mantelaufbaukörper 13 weist einen hier ebenfalls umfangsgeschlossen ausgebildeten und gegenüber dem Wurzelbereich 3 des Grundstifts 2 hervorspringenden Schulterbereich 14 auf. In allen Ausführungsbeispielen gemäß der Fig. 3 bis 7 handelt es sich um identisch ausgeformte Grundstifte 2 auf die in der nachfolgend noch geschilderten Art und Weise jeweils der Mantelkörper 13 aufgebracht wurde. In bevorzugten Ausgestaltungsformen, wie den hier gezeigten, besteht der Mantelaufbaukörper 13 abgesehen von Pigmenten zur Erhöhung der Opazität aus demselben zusammengesetzten Material 7 wie der Grundstift 2. Die Pigmente werden so gewählt, dass die Farbe des Mantelaufbaukörpers 13 der natürlichen Farbgebung von Dentin möglichst nahe kommt. Die Verbindung zwischen dem Mantelaufbaukörper 13 und dem Grundstift 2 erfolgt zusätzlich zum Formschluss noch durch Aushärten des organischen Bindemittels 8 des Mantelaufbaukörpers 13. Zusätzlich sind bei bevorzugten Ausgestaltungsformen auch die Glaskörner 9 des Grundstifts 2 und des Mantelaufbaukörpers 13 im Übergangsbereich zwischen Grundstift 2 und Mantelaufbaukörper 13 miteinander verschmolzen.

Der Bereich des Grundstiftes 2, welcher in den Ausgestaltungsformen der Fig. 3 bis 7 jeweils innerhalb des Mantelaufbaukörpers 13 angeordnet ist, also im Wesentlichen der Kronenbereich 5 des Grundstiftes 2, ist in den Fig. 3 bis 7 nur noch gestrichelt dargestellt, da er von außen in dargestellten Perspektiven nicht sichtbar ist. Eine Ausnahme hiervon bildet jeweils die Lichteinspeisefläche 10 des Grundstiftes 2. Diese sollte vom Mantelaufbaukörper 13 in einer von außen zugänglichen Art und Weise freigehalten werden, sodass Licht in die Lichteinspeisefläche 10 eingespeist und über die als Linse fungierende Verdickung 11 in den Wurzelbereich 13 und bevorzugt auch in die Schulterbereiche 14 weitergeleitet werden kann. Der Mantelaufbaukörper 13 ist, wie auch der Grundstift 2, bevorzugt monolithisch, also aus einem Guss ausgebildet. Auch im Mantelaufbaukörper 13 sind die Glaskörner 9 des zusammengesetzten Materials 7 bevorzugt nicht nur vom ausgehärteten organischen Bindemittel 8 sondern auch durch miteinander Verschmelzen miteinander verbunden. Der Mantelaufbaukörper 13 kann, soweit vorhanden, als eine Vergrößerung und/oder Formanpassung des Kronenbereichs 5 an den konkret zu behandelnden Zahntyp genutzt werden. So kann mit Hilfe des Mantelaufbaukörpers 13 quasi die Kontaktfläche des Endodontiestiftes 1 zur Zahnkronenverblendung 6 gegenüber dem Kronenbereich 5 des Grundstiftes 2 vergrößert werden. Die Zahnkronenverblendung 6 wird in solchen Ausführungsvarianten der Erfindung dann eben unter Zwischenschaltung des Mantelaufbaukörpers 13, also quasi indirekt, am Kronenbereich 5 des Grundstiftes 2 befestigt. Darüber hinaus kann der Mantelaufbaukörper 13 durch eine entsprechende farbliche Ausgestaltung auch zur Farbanpassung der aus der Zahnwurzel 4 herausragenden Teile des jeweiligen Endodontiestiftes 1 genutzt werden.

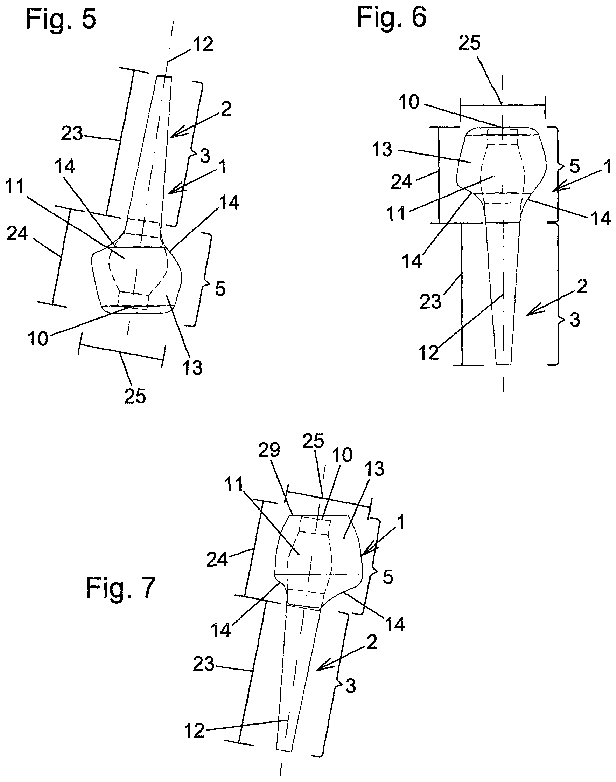

Fig. 3 zeigt ein Ausführungsbeispiel eines Endodontiestiftes 1, welcher speziell für einen oberen Frontzahn vorgesehen ist. Fig. 4 zeigt eine Ausführungsvariante eines Endodontiestiftes 1 für einen unteren Frontzahn. Die Fig. 5 und 6 zeigen Ausführungsvarianten von Endodontiestiften 1 für obere (Fig. 5) und untere (Fig. 6) vordere Backenzähne. Fig. 7 zeigt eine Ausführungsvariante für einen unteren hinteren Backenzahn. Für Backenzähne hat es sich als günstig herausgestellt, dass die Längsachse 12 des Wurzelbereichs 3 des Grundstiftes 2 in einem Winkel von günstigerweise 79° gegen eine obere Deckfläche 29 des Mantelaufbaukörpers 13 ausgerichtet ist.

Die Länge 23 des Wurzelbereichs 3 des Grundstiftes 2 liegt bevorzugt in einem Bereich von 11 mm bis 13mm, bevorzugt bei 12mm. Die Länge 24 des Kronenbereichs 5 des Grundstiftes 2 beträgt günstigerweise zwischen 3mm und 5mm, bevorzugt 4mm. Die Gesamtlänge 22 des Grundstiftes 2 ergibt sich entsprechend aus der Summe dieser beiden Werte. An seinem unteren, vom Kronenbereich 5 abgewandten Ende besitzt der Wurzelbereich 3 günstigerweise eine Breite 26 zwischen 1,0mm und 1,5mm. Bevorzugt handelt es sich bei diesem Ende um ein stumpfes also nicht spitzes Ende. Die Breite 27 an dem, dem Wurzelbereich 3 entgegengesetzten Ende des Kronenbereichs 5 des Grundstiftes 2 beträgt bevorzugt zwischen 1,8mm und 2,2mm, vorzugsweise 2mm. Bei Varianten mit Mantelaufbaukörper 13, wie sie beispielhaft in den Fig. 3 bis 7 dargestellt sind, kann die Länge 23 des Wurzelbereichs 3 auch etwas verkürzt gegenüber dem Grundstift 2 in einem Bereich von 9mm bis 11mm, vorzugsweise von 10mm, liegen. Die Breite 30 des Mantelaufbaukörpers 13 kann in bevorzugten Ausgestaltungsformen z.B. zwischen 4mm und 9mm variieren. Die Länge 31 des Mantelaufbaukörpers 13 kann z.B. ebenfalls zwischen 4mm und 10mm variieren. Die genannten Längen werden parallel zur Längsachse 12, die genannten Breiten rechtwinkelig dazu gemessen.

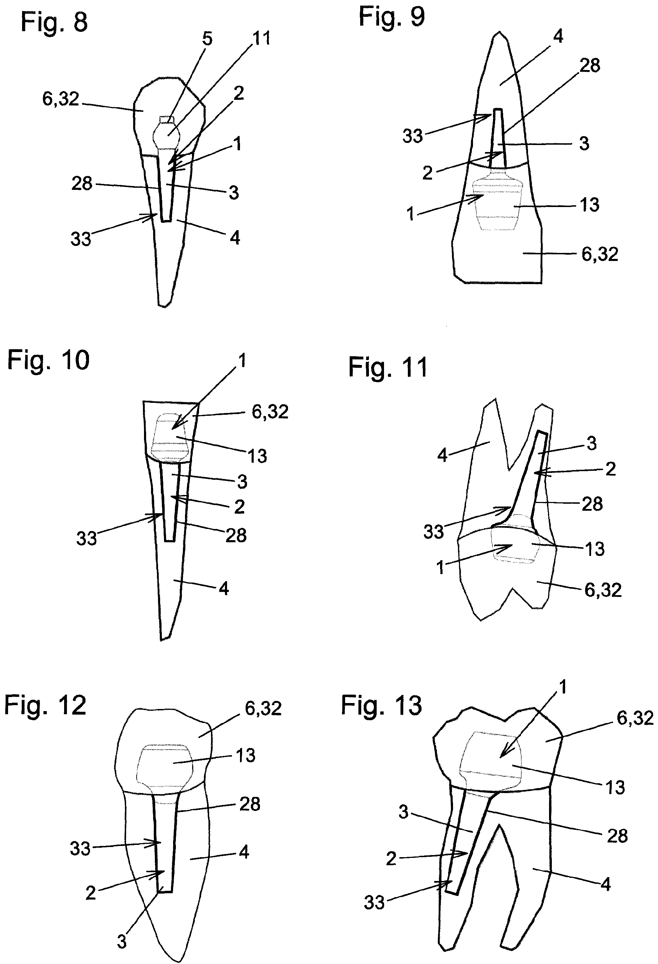

In den Fig. 8 bis 13 sind nun schematisiert verschiedene Zähne dargestellt, in welche die verschiedenen, erfindungsgemäßen Ausführungsbeispiele von Endodontiestiften 1 gemäß der Fig. 1 bis 7 appliziert worden sind. Von den Zähnen muss zumindest noch ein ausreichend großer Teil der natürlichen Zahnwurzel 4 vorhanden sein, damit in einem, vorzugsweise eingebohrten, Kanal 33 der natürlichen Zahnwurzel 4 der Wurzelbereich 3 des Endodontiestiftes 1 befestigt werden kann. Die ursprünglich vorhandene natürliche Zahnkrone 32 kann ganz oder teilweise fehlen. Die fehlenden Teile der Zahnkrone 32 sind in den Fig. 8 bis 13 durch entsprechende Zahnkronenverblendungen 6 also künstlich gefertigte Teile ersetzt. Die Zahnkronenverblendungen 6 sind am Endodontiestift 1 je nach erfindungsgemäßer Ausführung entweder direkt am Kronenbereich 5 oder indirekt, unter Zwischenschaltung eines Mantelaufbaukörpers 13 am Kronenbereich 5 befestigt. Die Zähne in den Fig. 8 bis 13 sind jeweils durchsichtig dargestellt, sodass man den Endodontiestift 1 im Inneren des natürlichen Zahnes sieht. In den schematisierten Darstellungen dieser Figuren ist auch offen gelassen, welche Teile der Zahnkrone 32 noch aus natürlicher Substanz bestehen und welche Teile durch eine Zahnkronenverblendung 6 ersetzt wurden. Dies kann, wie gesagt, je nach individuellem Anwendungsfall zwischen dem Totalersatz der natürlichen Substanz und dem Ersetzen relativ kleiner Teile der natürlichen Substanz variieren.

Fig. 8 zeigt die Verwendung des Endodontiestiftes 1 gemäß der Fig. 1 und 2, welcher ausschließlich aus dem Grundstift 2 besteht. Fig. 9 zeigt den Einsatz des Endodontiestiftes 1 gemäß Fig. 3, Fig. 10 den des Endodontiestiftes 1 gemäß Fig. 4. Bei den Fig. 9 und 10 handelt es sich jeweils um Frontzähne. Die Fig. 11 und 12 zeigen die Anordnung der Endodontiestifte aus den Fig. 5 und 6 jeweils in einem vorderen Backenzahn. Fig. 13 zeigt einen hinteren Backenzahn, bei dem der Endodontiestift 1 gemäß Fig. 7 appliziert wurde.

Dargestellt ist jeweils die natürliche Zahnwurzel 4, in die ein Kanal 33 eingebohrt wurde. Im Kanal 33 ist der jeweilige Endodontiestift 1 mittels des Befestigungsmaterials 28 einzementiert bzw. eingeklebt. Das Befestigungsmaterial 28 kann, bevor die Zahnkronenverblendung 6 aufgesetzt oder die Zahnkrone 32 komplettiert wird, durch Lichteinleitung in die jeweilige Lichteinspeisefläche 10 ausgehärtet werden, da der entsprechend lichtleitend ausgebildete Grundstift 2 des jeweiligen Endodontiestiftes 1 das zum Beschleunigen des Aushärtens des Befestigungsmaterials 28 benötigte Licht von der Lichteinspeisefläche 10 in den Wurzelbereich 3 und gegebenenfalls auch in den Schulterbereich 14 und somit direkt zum Befestigungsmaterial 28 leitet. Sobald der jeweilige Endodontiestift 1 entsprechend in der natürlichen Zahnwurzel 4 befestigt ist, kann anschließend auf den Kronenbereich 5 des Grundstiftes 2 bzw. soweit vorhanden, auf den Mantelaufbaukörper 13 des Endodontiestiftes 1 eine Zahnkronenverblendung 6 aufgebracht werden. Diese Zahnkronenverblendungen 6 sind an sich bekannt. Sie können, wie beim Stand der Technik bekannt, gefertigt und an dem jeweiligen Kronenbereich 5 bzw. Mantelaufbaukörper 13 durch an sich bekannte Mittel befestigt werden. Es kann sich aber auch bei der Zahnkronenverblendung 6 um an sich bekannte Veneers, also Zahnfrontverblendungskörper oder sogenannte Onlays, also künstliche Kauflächenprothesen handeln. Sofern noch Bereiche der natürlichen Zahnkrone 32 vorhanden sind, können diese aber auch nach Einsetzen des Endodontiestiftes 1 in die Zahnwurzel 4 mit beim Stand der Technik an sich bekannten Mitteln zur aufbauenden Ausbildung einer Zahnkronenverblendung 6 ergänzt und zu einer vollständigen Zahnkrone 32 wieder aufgebaut werden. Der Kronenbereich 5, bzw. soweit vorhanden der Mantelaufbaukörper 13, ist dann in der natürlichen Zahnkrone 32 angeordnet und dient zu deren Stabilisierung und als Befestigungspunkt für die künstlich hinzugefügten Teile der Zahnkrone 32.

Der Vollständigkeit halber wird noch darauf hingewiesen, dass, wie in den Fig. 8 bis 13 auch dargestellt, es durchaus sein kann, dass der Wurzelbereich 3 des Grundstiftes 2 ein Stück weit auch in die Zahnkronenverblendung 6 bzw. in die Zahnkrone 32 hineinragt. Andersherum kann es in der Praxis auch gut sein, dass der Mantelaufbaukörper 13 oder der Kronenbereich 5 und insbesondere auch der Schulterbereich 14 ein Stück weit in die natürliche Zahnwurzel 4 versenkt angeordnet werden.

Die Fig. 11 und 13 zeigen, dass bei den vorderen und hinteren Backenzähnen, bei denen ja auch mehrere natürliche Zahnwurzeln 4 vorhanden sein können, in der Regel nur in einer Zahnwurzel 4 der entsprechende Wurzelbereich 3 des Grundstiftes 2 bzw. des Endodontiestiftes 1 angeordnet wird.

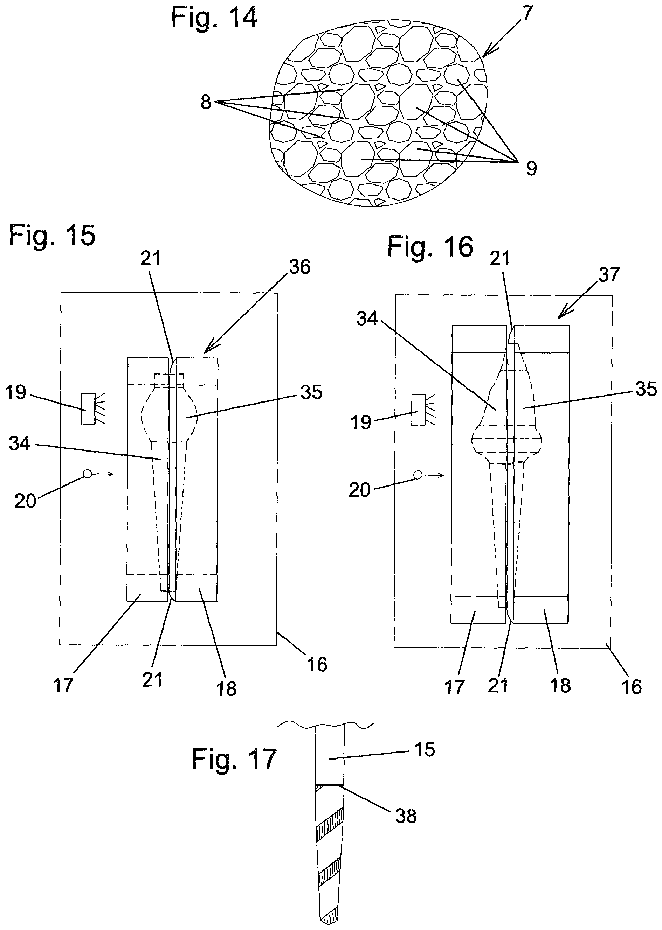

Fig. 14 zeigt schematisiert und stark vergrößert das zusammengesetzte Material 7 des Grundstiftes 2 wie auch des Mantelaufbaukörpers 13. Man sieht die sehr enge Packung der Glaskörner 9 mit dem dazwischen angeordneten organischen Bindemittel 8. In bevorzugten Ausgestaltungsformen sind die Glaskörner 9 aber nicht nur mittels des organischen Bindemittels 8 zusammengehalten sondern an ihren Oberflächen auch miteinander verschmolzen. Die eingangs genannten zusätzlich möglichen Füllstoffe wie Zinkoxid, Pigment, Katalysator und/oder Additive sind in Fig. 14 nicht dargestellt, da sie in der Regel auch nur sehr geringe Volumenanteile am gesamten zusammengesetzten Material 7 haben.

Nachfolgend wird ein mögliches Verfahren zur Herstellung erfindungsgemäßer Endodontiestifte 1 beispielhaft erläutert. Einzelne Schritte des Verfahrens, wie z.B. das Polymerisieren mittels der LED-Lampe 19 und/oder die Laserbestrahlung zum Verschmelzen der Glaskörner 9 können auch mit anderen Verfahrensschritten als den nachfolgend geschilderten kombiniert werden.

Zunächst müssen die einzelnen Bestandteile des zusammengesetzten Materials 7 also vorrangig das organische Bindemittel 8 und die, bevorzugt vorab silanisierten, Glaskörnern 9 und gegebenenfalls weitere Füllstoffe wie Zinkoxid, Katalysatoren und/oder Additive miteinander vermischt werden. Alternativ kann auch, wie eingangs bereits erläutert, auf im Handel vorhandene fertige Mischungen des zusammengesetzten Materials 7 zurückgegriffen werden.

Zunächst wird nun die Herstellung eines Grundstiftes 2 anhand von Fig. 15 erläutert.

Da der Grundstift 2 in bevorzugten Ausgestaltungsformen lichtleitend ausgebildet sein soll, muss er eine entsprechend hohe Transparenz bzw. Transluzenz aufweisen. Entsprechend weist das zusammengesetzte Material 7 für den Grundstift 2 auch keine oder nur sehr geringe Mengen an Pigmenten, also Farbstoffen auf. Das fertig gemischte bzw. aus dem Handel erhältliche zusammengesetzte Material 7 wird nun zunächst auf eine Temperatur von 80° C erwärmt und in einem Reinraum 16 in eine offene Form 36, im gezeigten Ausführungsbeispiel in deren zweite Formhälfte 18, in ausreichender Menge eingefüllt. Die zweite Formhälfte 18 besteht bevorzugt aus Metall, insbesondere aus Stahl. Die den Formhohlraum 35 dieser Formhälfte 18 begrenzende Oberfläche kann spiegelnd ausgeführt sein. Anschließend wird die erste Formhälfte 17, welche bevorzugt aus Glas oder einem anderen entsprechend lichtdurchlässigen Material besteht, auf die zweite Formhälfte 18 aufgepresst, sodass die aus den Formhälften 17 und 18 bestehende Form 36 geschlossen ist. Die die Form des herzustellenden Grundstiftes 2 vorgebenden Hohlräume 34 und 35 in den beiden Formhälften 17 und 18 müssen dabei vollständig mit zusammengesetztem Material 7 gefüllt sein. Überschüssiges Material 7 wird beim Schließen der Formhälften 17 und 18 zwischen diesen herausgepresst. Die in diesem Ausführungsbeispiel gezeigten Anfasungen 21 sorgen beim Schließen der Formhälften 17 und 18 dafür, dass am Grundstift 2 keine Grate entstehen, welche anschließend entfernt werden müssten.

Alternativ hierzu könnte man das zusammengesetzte Material 7 natürlich auch in eine vorab geschlossene Form mittels Spritzguss oder dergleichen einbringen.

Die gefüllte und geschlossene Form wird nun in bevorzugten Ausgestaltungsformen des Verfahrens auf eine Temperatur von 300° C aufgeheizt und zumindest eine halbe Stunde auf dieser Temperatur gehalten. Hierdurch findet ein temperaturbedingter Polymerisationsprozess zum Aushärten des organischen Bindemittels 8 im zusammengesetzten Material 7 statt. Um die Polymerisation bzw. das Aushärten des organischen Bindemittels 8 weiter zu fördern, ist im gezeigten Ausführungsbeispiel eine LED-Lampe 19 vorgesehen. Das Licht der LED-Lampe 19, welches bevorzugt in einem Wellenlängenbereich von 395 bis 480 Nanometer, besonders bevorzugt von 450 bis 480 Nanometer liegt, gelangt durch die lichtdurchlässige erste Formhälfte 17 zum zusammengesetzten Material 7 in den Formhohlräumen 34 und 35 und fördert weiter die Polymerisation bzw. das Aushärten des organischen Bindemittels 8. Die LED-Lampe 19 kann entweder so groß ausgebildet sein, dass sie die gesamte erste Formhälfte 17 abdeckt. Genauso gut ist es natürlich auch möglich, die LED-Lampe 19 entlang der ersten Formhälfte 17 zu bewegen, um so den gesamten Formhohlraum 34 bzw. 35 gleichmäßig mit LED-Licht zu beaufschlagen.

Anschließend an diesen Aushärtungs- bzw. Polymerisationsprozess des organischen Bindemittels 8 wird das zusammengesetzte Material 7 in den Formhohlräumen 34 und 35 anschließend noch einer Laserbehandlung unterzogen, um die Glaskörner 9 des zusammengesetzten Materials 7 miteinander zu verschmelzen. Hierfür wird das zusammengesetzte Material 7 in den Formhohlräumen 34 und 35 durch die erste Formhälfte 17 hindurch mittels der Laserlichtquelle 20 mit Laserlicht beaufschlagt. Die Intensität und Bestrahlungsdauer wird so eingestellt, dass das Laserlicht durch das gesamte zusammengesetzte Material 7 hindurchdringt und so die Glaskörner 9 im zusammengesetzten Material 7 im gesamten sich in den Formhälften 17 und 18 befindenden Grundstift 2 miteinander verschmolzen werden. Die Laserlichtquelle 20 kann hierzu in geeigneter Art und Weise über die gesamte erste Formhälfte 17 bzw. den gesamten Bereich der Formhohlräume 34 und 35 hinweggeführt werden. Nach Abschluss der Laserbehandlung kann der fertiggestellte Grundstift 2 aus den Formhälften 17 und 18 entnommen werden. Durch das Aushärten des organischen Bindemittels 8 und das anschließende Verschmelzen der Glaskörner 9, insbesondere an deren Oberflächen, wird bevorzugt ein monolithischer Grundstift 2 hergestellt. Soll der herzustellende erfindungsgemäße Endodontiestift 1 ausschließlich aus dem Grundstift 2 bestehen, so ist der Herstellungsprozess damit abgeschlossen, wobei natürlich Nachbehandlungen z.B. der Oberfläche nicht ausgeschlossen sind.

Soll der erfindungsgemäße Endodontiestift 1 hingegen auch einen Mantelaufbaukörper 13 aufweisen, so wird der fertige Grundstift 2 in eine in Fig. 16 gezeigte zweite Form 37 eingelegt. Auch dies geschieht in einem Reinraum 16 bei geöffneten Formhälften 17 und 18. Das für den Mantelaufbaukörper 13 vorgesehene zusammengesetzte Material 7, welches die entsprechenden Pigmente enthält und ansonsten dasselbe Ausgangsmaterial wie beim Grundstift 2 sein kann, wird wie bei der Herstellung des Grundstiftes zunächst auf eine Temperatur von 80° C gebracht und dann in die neben dem Grundstift 2 verbleibenden Bereiche der Formhohlräume 34 und 35 der zweiten Form 37 eingefüllt. Im Anschluss daran werden die beiden Formhälften 17 und 18 so aufeinandergedrückt, dass die Form 37 geschlossen wird. Die Anfasungen 21 haben beim Schließen der Form 37 dieselbe Wirkung wie bei der Form 36. Das anschließende Aushärten des organischen Bindemittels 8 sowie die bevorzugt vorgesehene Laserbehandlung zum Verschmelzen der Glaskörner 9 im Mantelaufbaukörper 13 kann dann anschließend analog zu den entsprechenden Verfahrensschritten bei der Herstellung des Grundstiftes 2 durchgeführt werden. Aufgrund der lichtleitenden Eigenschaften des Grundstiftes 2 kommt es dabei zu keinen Abschattungen, sodass sowohl das LED-Licht als auch das Laserlicht den gesamten Mantelaufbaukörper 13 durchdringen können und so auch ein in sich monolithischer Mantelaufbaukörper 13 geschaffen werden kann. Bei diesen Verfahrensschritten wird auch automatisch die entsprechende Verbindung des Mantelaufbaukörpers 13 mit dem Grundstift 2 erreicht. Nach Abschluss dieser, wie bei der Herstellung des Grundstiftes 2 durchzuführenden Verfahrensschritte kann der fertige Endodontiestift 1 aus den Formhälften 17 und 18 dieser zweiten Form 37 entnommen werden. Auch hier sind natürlich noch gewisse Nachbearbeitungen z.B. der Oberfläche des Mantelaufbaukörpers 13 möglich, wenn auch nicht unbedingt notwendig.

Fig. 17 zeigt schematisiert noch einen Bohrer 15, dessen Form, z.B. durch entsprechend konische Ausformung, an die des Wurzelbereichs 3 des jeweiligen Grundstiftes 2 des Endodontiestiftes 1 angepasst ist. Hierdurch wird erreicht, dass durch Verwendung dieses Bohrers 15 ein zu der Form des Wurzelbereichs 3 optimal passender Kanal 33 in der jeweiligen Zahnwurzel 4 geschaffen wird. Der Bohrer 15 kann zur Sicherstellung der optimalen Tiefe des Kanals 33 eine bevorzugt optisch erkennbare, z.B. farbige, Markierung 38 und/oder auch einen entsprechenden, hier nicht dargestellten Längsanschlag aufweisen.

Es kann in diesem Zusammenhang vorgesehen sein, dass ein oder mehrere Endodontiestifte 1 in einem Set zusammen mit entsprechend passenden Bohrern 15 verkauft werden. Dieses Set kann zusätzlich auch das entsprechend passende Befestigungsmaterial 28 mit umfassen.

**Legende zu den Hinweisziffern:**

| | | | |
|---|---|---|---|
| 1 | Endodontiestift | 20 | Laserlichtquelle |
| 2 | Grundstift | 21 | Anfasung |
| 3 | Wurzelbereich | 22 | Gesamtlänge |
| 4 | Zahnwurzel | 23 | Länge |
| 5 | Kronenbereich | 24 | Länge |
| 6 | Zahnkronenverblendung | 25 | Breite |
| 7 | zusammengesetztes Material | 26 | Breite |
| 8 | organisches Bindemittel | 27 | Breite |
| 9 | Glaskorn | 28 | Befestigungsmaterial |
| 10 | Lichteinspeisefläche | 29 | obere Deckfläche |
| 11 | Verdickung | 30 | Breite |
| 12 | Längsachse | 31 | Länge |
| 13 | Mantelaufbaukörper | 32 | Zahnkrone |
| 14 | Schulterbereich | 33 | Kanal |
| 15 | Bohrer | 34 | Formhohlraum |
| 16 | Reinraum | 35 | Formhohlraum |
| 17 | erste Formhälfte | 36 | erste Form |
| 18 | zweite Formhälfte | 37 | zweite Form |
| 19 | LED-Lampe | 38 | Markierung |

## Patentansprüche

1. Endodontiestift (1) mit einem Grundstift (2), wobei der Grundstift (2) einen Wurzelbereich (3) zur Befestigung des Grundstifts (2) in einer natürlichen Zahnwurzel (4) und einen Kronenbereich (5) zur Befestigung einer Zahnkronenverblendung (6) am Endodontiestift (1) und/oder zur Anordnung in einer natürlichen Zahnkrone (32) aufweist und der Grundstift (2) aus einem zusammengesetzten Material (7) besteht, welches zumindest ein organisches Bindemittel (8) und Glaskörner (9) als Füllstoff aufweist, **dadurch gekennzeichnet, dass** die Glaskörner (9) miteinander verschmolzen sind.

2. Endodontiestift (1) nach Anspruch 1, **dadurch gekennzeichnet, dass** die Glaskörner (9) Bariumglas und/oder Strontiumglas aufweisen oder daraus bestehen und/oder dass die Glaskörner (9) eine silanisierte Oberfläche aufweisen.

3. Endodontiestift (1) nach Anspruch 1 oder 2, **dadurch gekennzeichnet, dass** das organische Bindemittel (8) zumindest ein Methacrylat aufweist oder ist und/oder dass das zusammengesetzte Material (7) als Füllstoff zusätzlich Zinkoxid und/oder zumindest ein Pigment und/oder zumindest einen Katalysator und/oder zumindest ein Additiv aufweist.

4. Endodontiestift (1) nach einem der Ansprüche 1 bis 3, **dadurch gekennzeichnet, dass** der Grundstift (2) im Kronenbereich (5) eine Lichteinspeisefläche (10) aufweist und zur Weiterleitung von über die Lichteinspeisefläche (10) eingespeistem Licht in den Wurzelbereich (3) zumindest bereichsweise, vorzugsweise vollständig, lichtleitend ausgebildet ist.

5. Endodontiestift (1) nach einem der Ansprüche 1 bis 4, **dadurch gekennzeichnet, dass** der Grundstift (2), vorzugsweise dessen Kronenbereich (5), einen, vorzugsweise eine Verdickung (11) des Grundstifts (2) ausbildenden, Linsenkörper aufweist.

6. Endodontiestift (1) nach einem der Ansprüche 1 bis 5, **dadurch gekennzeichnet, dass** der Wurzelbereich (3) des Grundstiftes (2) als ein sich in Richtung weg vom Kronenbereich (5) verjüngender Konus ausgebildet ist und/oder dass der Wurzelbereich (3) als ein bezüglich einer Längsachse (12) des Grundstiftes (2) rotationssymmetrischer Körper ausgebildet ist.

7. Endodontiestift (1) nach einem der Ansprüche 1 bis 6, **dadurch gekennzeichnet, dass** der Endodontiestift (1) zusätzlich zum Grundstift (2) einen Mantelaufbaukörper (13) aufweist, welcher, vorzugsweise ausschließlich, den Kronenbereich (5) des Grundstiftes (2), vorzugsweise in sich umfangsgeschlossen, ummantelt, wobei vorzugsweise vorgesehen ist, dass der Mantelaufbaukörper (13) eine höhere Opazität als der Grundstift (2) aufweist.

8. Endodontiestift (1) nach Anspruch 7, **dadurch gekennzeichnet, dass** der Mantelaufbaukörper (13), vorzugsweise umfangsgeschlossen, einen gegenüber dem Wurzelbereich (3) des Grundstifts (2) hervorspringenden Schulterbereich (14) des Endodontiestiftes (1) ausbildet und/oder dass der Mantelaufbaukörper (13) abgesehen von Pigmenten aus demselben zusammengesetzten Material (7) wie der Grundstift (2) besteht oder ein solches aufweist.

9. Endodontiestift (1) nach einem der Ansprüche 1 bis 8, **dadurch gekennzeichnet, dass** der Grundstift (2) und/oder der gegebenenfalls vorhandene Mantelaufbaukörper (13) in sich monolithisch ausgebildet ist bzw. sind und/oder dass die Glaskörner (9) im gesamten Grundstift (2) und/oder im gesamten gegebenenfalls vorhandenen Mantelaufbaukörper (13) miteinander verschmolzen sind.

10. Set mit zumindest einem Endodontiestift (1) nach einem der Ansprüche 1 bis 9 und zumindest einem Bohrer (15), wobei die Form des Bohrers (15) an die Form des Wurzelbereichs (3) des Grundstifts (2) des Endodontiestiftes (1) angepasst ist.

## Claims

1. An endodontic pin (1) having a base pin (2), wherein the base pin (2) has a root portion (3) for fastening the base pin (2) in a natural tooth root (4) and a crown portion (5) for fastening a tooth crown facing (6) to the endodontic pin (1) and/or for arranging in a natural tooth crown (32), and the base pin (2) is composed of a composite material (7) having at least one organic binder (8), and the composite material (7) has glass particles (9) as filler, **characterized in that** the glass particles (9) are fused together.

2. The endodontic pin (1) as claimed in claim 1, **characterized in that** the glass particles (9) have or are composed of barium glass and/or strontium glass, and/or **in that** the glass particles (9) have a silanized surface.

3. The endodontic pin (1) as claimed in claim 1 or 2, **characterized in that** the organic binder (8) has or is at least one methacrylate, and/or **in that** the composite material (7) additionally has, as filler, zinc oxide and/or at least one pigment and/or at least one catalyst and/or at least one additive.

4. The endodontic pin (1) as claimed in one of claims 1 through 3, **characterized in that** the base pin (2) has a light admission surface (10) in the crown portion (5) and, in order that light introduced via the light admission surface (10) is conveyed onward into the root portion (3), is designed to be light-transmitting at least in part, preferably completely.

5. The endodontic pin (1) as claimed in one of claims 1 through 4, **characterized in that** the base pin (2), preferably the crown portion (5) thereof, has a lens body that preferably forms a thickening (11) of the base pin (2).

6. The endodontic pin (1) as claimed in one of claims 1 through 5, **characterized in that** the root portion (3) of the base pin (2) is designed as a cone tapering in a direction away from the crown portion (5), and/or **in that** the root portion (3) is designed as a body that is rotationally symmetrical with respect to a longitudinal axis (12) of the base pin (2).

7. The endodontic pin (1) as claimed in one of claims 1 through 6, **characterized in that** the endodontic pin (1) has, additionally to the base pin (2), an attachment casing (13) which envelops, preferably exclusively, the crown portion (5) of the base pin (2), preferably in a circumferentially closed manner, wherein provision is preferably made that the attachment casing (13) has a higher opacity than the base pin (2).

8. The endodontic pin (1) as claimed in claim 7, **characterized in that** the attachment casing (13) forms, preferably in a circumferentially closed manner, a shoulder portion (14) of the endodontic pin (1) protruding relative to the root portion (3) of the base pin (2), and/or **in that** the attachment casing (13), apart from pigments, is composed of the same composite material (7) as the base pin (2) or has such a material.

9. The endodontic pin (1) as claimed in one of claims 1 through 8, **characterized in that** the base pin (2) and/or the optionally present attachment casing (13) are/is of an inherently monolithic configuration, and/or **in that** the glass particles (9) are fused together in the entire base pin (2) and/or in the entire optionally present attachment casing (13).

10. A kit having at least one endodontic pin (1) as claimed in one of claims 1 through 9 and having at least one drill (15), wherein the shape of the drill (15) is adapted to the shape of the root portion (3) of the base pin (2) of the endodontic pin (1).

## Revendications

1. Tenon endodontique (1) avec une tige de base (2), la tige de base (2) présentant une zone racinaire (3) pour fixer la tige de base (2) dans une racine dentaire naturelle (4), et une zone de couronne (5) pour fixer un placage de couronne dentaire (6) sur le tenon endodontique (1) et/ou pour être placée dans une couronne dentaire naturelle (32), et la tige de base (2) étant constituée d'un matériau composite (7) qui comprend au moins un liant organique (8) et des grains de verre (9) en tant que charge, **caractérisé en ce que** les grains de verre (9) sont fusionnés ensemble.

2. Tenon endodontique (1) selon la revendication 1, **caractérisé en ce que** les grains de verre (9) comprennent ou sont constitués de verre de baryum et/ou de verre de strontium, et/ou **en ce que** les grains de verre (9) ont une surface silanisée.

3. Tenon endodontique (1) selon la revendication 1 ou 2, **caractérisé en ce que** le liant organique (8) comprend ou consiste en au moins un méthacrylate, et/ou **en ce que** le matériau composite (7) comprend en tant que charge en outre de l'oxyde de zinc et/ou au moins un pigment et/ou au moins un catalyseur et/ou au moins un additif.

4. Tenon endodontique (1) selon l'une des revendications 1 à 3, **caractérisé en ce que** la tige de base (2) présente une surface d'alimentation en lumière (10) dans la zone de couronne (5) et est conçue conductrice de lumière au moins par zones, de préférence entièrement, pour retransmettre dans la zone de la racine (3) la lumière introduite par la surface d'alimentation en lumière (10).

5. Tenon endodontique (1) selon l'une des revendications 1 à 4, **caractérisé en ce que** la tige de base (2), de préférence sa zone de couronne (5), présente un corps en forme de lentille, formant de préférence un épaississement (11) de la tige de base (2).

6. Tenon endodontique (1) selon l'une des revendications 1 à 5, **caractérisé en ce que** la zone racinaire (3) de la tige de base (2) est réalisée sous la forme d'un cône se rétrécissant en s'éloignant de la zone de couronne (5), et/ou **en ce que** la zone racinaire (3) est réalisée sous la forme d'un corps ayant une symétrie de rotation par rapport à un axe longitudinal (12) de la tige de base (2).

7. Tenon endodontique (1) selon l'une des revendications 1 à 6, **caractérisé en ce que** le tenon endodontique (1) présente, en plus de la tige de base (2), une structure d'enveloppe (13) qui entoure, de préférence exclusivement, la zone de couronne (5) de la tige de base (2), de préférence refermée sur elle-même sur sa circonférence, la structure d'enveloppe (13) présentant de préférence une opacité plus élevée que la tige de base (2).

8. Tenon endodontique (1) selon la revendication 7, **caractérisé en ce que** la structure d'enveloppe (13), de préférence refermée sur elle-même sur sa circonférence, forme une zone d'épaulement (14) du tenon endodontique (1) qui saille par rapport à la zone racinaire (3) de la tige de base (2), et/ou **en ce que** la structure d'enveloppe (13), à l'exception de pigments, est constituée du même matériau composite (7) que la tige de base (2) ou est munie de celui-ci.

9. Tenon endodontique (1) selon l'une des revendications 1 à 8, **caractérisé en ce que** la tige de base (2) et/ou la structure d'enveloppe (13) éventuellement présente est/sont monolithique(s) en soi, et/ou **en ce que** les grains de verre (9) sont fusionnés ensemble dans la totalité de la tige de base (2) et/ou dans la totalité de la structure d'enveloppe (13) éventuellement présente.

10. Set comprenant au moins un tenon endodontique (1) selon l'une des revendications 1 à 9 et au moins un foret (15), la forme du foret (15) étant adaptée à la forme de la région racinaire (3) de la tige de base (2) du tenon endodontique (1).
